# EUROPEAN PATENT SPECIFICATION

(11) **EP 2 377 505 B1**
(45) Date of publication and mention of the grant of the patent: **30.11.2016**
(21) Application number: 09836445.8
(22) Date of filing: 09.04.2009
(51) Int. Cl.: B01F 5/04, B01F 3/08, A61J 3/07

(54) **METHOD FOR PRODUCING LIPOSOMAL DRUGS AND A DEVICE FOR PRODUCING A LIPOSOME**
VERFAHREN ZUR HERSTELLUNG LIPOSOMALER ZUSAMMENSETZUNG UND VORRICHTUNG ZUR HERSTELLUNG EINES LIPOSOMS
PROCÉDÉ DE FABRICATION DE PRÉPARATIONS LIPOSOMALES ET DISPOSITIF DE FABRICATION DE LIPOSOMES

(30) Priority: 29.12.2008 RU 2008151923
(43) Date of publication of application: 19.10.2011
(73) Proprietor: Grebennikov, Evgeny Petrovich, Moscow 105425 (RU)
(72) Inventor: Grebennikov, Evgeny Petrovich, Moscow 105425 (RU)
(74) Representative: AMMANN PATENTANWÄLTE AG BERN
(86) International application number: PCT/RU2009/000172
(87) International publication number: WO 2010/077167

(56) References cited:
- EP-A1- 0 399 041
- WO-A1-99/42545
- WO-A1-2004/101123
- WO-A2-2006/096571
- GB-A- 2 145 107
- US-A- 3 251 550
- US-A1- 2005 175 683
- KOVALNOGOV N.N.: 'Raschet techeniya i coprotivleniya treniya potoka v soplakh zavalya.' POSOBIE K KURSOVOY RABOTE PO GIDROGAZODINAMIKE, ULYANOVSK, [Online] 2001, pages 1 - 21, XP008172238 Retrieved from the Internet: <URL:http://venec. ulstu.ru/lib/2002/1/Kovalqnogov.pdf>

## Description

The present invention relates to a method and a device for producing liposomal drugs in an aerosol stream.

### FIELD OF THE INVENTION

The invention relates to the field of applied biotechnology and may be used in medicine, cosmetology, veterinary medicine, crop science, etc., to increase the efficiency of the preparation of liposomal drugs in the form of an aerosol stream.

### BACKGROUND OF THE INVENTION

A method is known from prior art for producing liposomal drugs in the form of an aerosol stream, including mixings of an aqueous medium, fed into a mixing chamber, with a lipid component - a solution of lipids in an organic solvent, and the subsequent formation of an aerosol stream by spraying from a nozzle with application to a surface to be treated (GB 2145107 A, 1985). However, the process of the preparation of liposomal vesicles by simple mixing of the lipid component - a solution of lipids, with an aqueous medium is insufficiently efficient.

A device is also known for producing liposomes in the form of an aerosol stream, including vessels with an aqueous medium and a lipid component - a solution of lipids in a water-soluble organic solvent, that are connected to a mixer with a spray nozzle at the outlet. (GB 2145107 A, 1985). In that device both components are fed into the mixer from the respective vessels under pressure created in the vessels by a propellant (a neutral gas); this complicates the device and does not ensure effective mixing of the components and a high degree of spraying - dispersion of the mixture formed.

### SUMMARY OF THE INVENTION

The invention is directed toward increasing the efficiency of the production of liposomal drugs in the form of an aerosol stream and the creation of a simple and efficient device for producing liposomes.

Such method is defined in claim 1, and a corresponding device in claim 3.

The solution of the stated problem is achieved by the fact that according to the invention in the method for producing liposomes that includes mixing of an aqueous medium, fed under pressure into a mixing chamber, with a lipid component - a solution of lipids in an organic solvent - and the subsequent formation of an aerosol stream by spraying from a nozzle with application to a surface to be treated, the mixing is accomplished by an ejector by means of the suction induced introduction of the lipid component - a solution of lipids in an organic solvent - into the mixing chamber of the ejector made in the form of a Laval nozzle by means of the energy of the jet of aqueous medium flowing out of the inlet nozzle of the ejector, which creates rarefaction in the narrowing part - the convergent tube - of the mixing chamber, with simultaneous dispersion and homogenization in the expanding part - the divergent tube - of the mixing chamber, wherein an aerosol stream of liposomes is formed at the outlet upon spraying from the expanding part of the divergent tube of the mixing chamber.

At the same time, the aqueous medium and/or the lipid component - a solution of lipids in an organic solvent - contain a biologically active substance.

In addition, the solution of the stated problem is achieved by the fact that in the device for producing liposomes, that includes a vessel with an aqueous medium and a vessel with a lipid component - a solution of lipids in a water-soluble organic solvent - which vessels are connected to a mixer with a spray nozzle at the outlet, according to the invention a pump is included between the vessel with the aqueous medium and the mixer, wherein the mixer is made in the form of an ejector, the central inlet active flow nozzle of which is connected with the outlet pipe of the pump, and the mixing chamber is made in the form of a Laval nozzle, to the convergent tube of which is connected the vessel with the lipid component and the divergent tube of which is a spray nozzle.

The construction of the mixer in the form of an ejector with a mixing chamber made in the form of a Laval nozzle with narrowing and expanding parts provides with simplicity of design ejection (suction) of the lipid component into the mixing chamber by means of the energy of the jet of aqueous medium flowing out of the central inlet nozzle of the ejector that creates rarefaction in the narrowing part (convergent tube), while, due to the hydrodynamic action, intense dispersion and homogenization of the mixture of components flowing through at high velocity takes place in the expanding part (divergent tube), and upon outflow from the divergent tube, as from a spray nozzle, the efficient formation takes place of a flow of finely dispersed drops containing a solution of a biologically active substance with a lipid envelope, forming a stable bilayer membrane - a vesicle (liposome).

The invention will be further explained by preferred exemplary execution manners and embodiments with reference to the Figure.

### BRIEF DESCRIPTION OF THE DRAWINGS

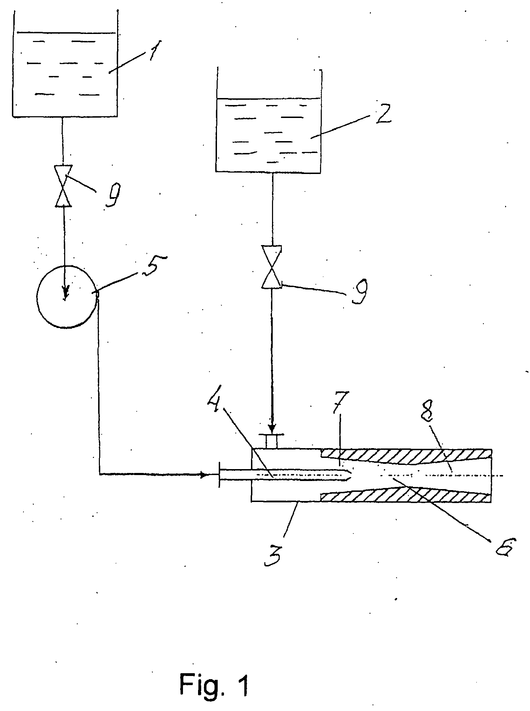

In Fig. 1, the device for producing liposomal drugs is represented schematically.

### VARIANTS OF EMBODIMENT OF THE INVENTION

The device for producing liposomal drugs contains a vessel 1, filled with aqueous medium that can contain a biologically active substance; a vessel 2, with a lipid component - a solution of lipids in a water-soluble organic solvent, that also can contain a biologically active substance; a mixer - ejector 3; a central active flow nozzle 4, is connected with the outlet pipe of a pump 5 included between vessel 1 and ejector 3. Mixing chamber 6 of ejector 3 is made in the form of a Laval nozzle, the convergent tube 7 of which is connected with the vessel 2 with the lipid component, and the divergent tube 8 of the Laval nozzle of mixing chamber 6 is a spray nozzle. Control valves 9 are mounted in the main pipelines connecting vessels 1 and 2 with ejector 3.

The method of producing liposomal drugs in the form of an aerosol stream is implemented in the following manner.

The lipid component is a solution of phospholipids (individual phospholipids or a mixture of them, produced from plant, animal, or biotechnological raw material) in an organic water-soluble solvent (ethanol, propanol, benzyl alcohol, hexane, methanol, chloroform, ether, etc.) with a concentration no less than 0.5%, that can contain a biologically active substance, is poured into vessel 2, and vessel 1 is filled with an aqueous medium, for example an aqueous solution of a biologically active substance. When the aqueous medium is delivered from vessel 1 into the central nozzle 4 of ejector 3 by pump 5, the jet of out-flowing active flow creates rarefaction in the convergent tube 7 of the Laval nozzle - mixing chamber 6 of ejector 3 - due to which ejection (suction) of the lipid component - the phospholipid solution from vessel 2 - takes place. In the divergent tube 8, the lipid component - the phospholipid solution, is actively mixed with the flow of the aqueous medium with the formation of a disperse homogeneous mixture of components, upon the spraying of which an aerosol stream of liposomes - finely dispersed drops containing a solution of a biologically active substance - with a lipid envelope forming a stable bilayer membrane - a vesicle (liposome) - is formed at the outlet of the divergent tube 8 of the Laval nozzle - mixing chamber 6 - as from a spray nozzle. The ratio of the flow rates of the components is controlled by means of valves 9. The aerosol stream formed of the aqueous suspension of liposomes containing the biologically active substance, is delivered from the divergent tube 8 directly onto the surface to be treated, for example the skin.

### Example 1.

To prepare the lipid component, a phospholipid extract (for example, Lipofolk) is dissolved in an organic water-soluble solvent (for example, ethanol - 70% ethyl alcohol) until a 10% concentration of phospholipids is obtained and is mixed in a 1:1 ratio with a biologically active substance: an alcohol (in 70% ethyl alcohol) tincture of calendula - a preparation with antiseptic and anti-inflammatory properties, prepared from dry flowers in a 1:10 ratio. The lipid component obtained is poured into vessel 2, and vessel 1 is filled with distilled water. The aerosol stream of the aqueous suspension of liposomes that contain the biologically active substance - calendula (8-10% extract of calendula) - that is formed as a result of the ejection mixing, is directed from the divergent tube 8 of the Laval nozzle - mixing chamber 6 - as from a spray nozzle onto the surface to be treated, for example, the skin.

### Example 2.

A lipid solution is prepared by dissolving a phospholipid extract (for example, Lipofolk) in an organic water-soluble solvent (for example, ethanol - 70% ethyl alcohol) until a 10% concentration of phospholipids is obtained and is poured into vessel 2. Vessel 1 is filled with an aqueous medium (an aqueous solution of a biologically active substance), for example, an aqueous-alcoholic tincture of mountain arnica flowers in a 40% alcohol solution (extraction modulus 1:15). The aerosol stream that is formed as a result of the ejection mixing of the aqueous suspension of liposomes, and that contains the biologically active substance - extract of mountain arnica, which improves local microcirculation and blood supply - promotes lymph drainage, maintains the tonus of veins, and removes edema of the legs - is directed from the divergent tube 8 of the Laval nozzle - mixing chamber 6 - as from a spray nozzle, onto the surface to be treated, for example the skin.

### Example 3.

To prepare the lipid component, a phospholipid extract (for example, Lipofolk) is dissolved in an organic water-soluble solvent (for example, ethanol - 70% ethyl alcohol) until a 10% concentration of phospholipids is obtained and is mixed in a 1:1 ratio with a biologically active substance: a mixture of an alcoholic extract of arnica (an aqueous-alcoholic tincture of mountain arnica flowers in a 40% alcohol solution) and of camomile (an aqueous-alcoholic tincture of wild camomile flowers in a 40% alcohol solution) in a 1:1 ratio with the phospholipids (1:2). The lipid component obtained is poured into vessel 2. Vessel 1 is filled with an aqueous medium (an aqueous solution of a biologically active substance), containing a mixture in a 1:1 ratio of an aqueous extract of flowers of wild camomile 1:20 in a water bath and an aqueous extract of flowers of mountain arnica 1:20 in a water bath. The aerosol stream that is formed as a result of the ejection mixing of the aqueous suspension of liposomes and that contains the mixture of biologically active substances - camomile and arnica, which exerts a stimulating action on the processes of skin regeneration - is directed from the divergent tube 8 of the Laval nozzle - mixing chamber 6 - as from a spray nozzle, onto the skin surface to be treated.

## Claims

1. A method for producing liposomal drugs in an aerosol stream, comprising:
mixing of an aqueous medium (1), fed under pressure into a mixing chamber (6), with a lipid component (2), the lipid component being a solution of lipids in an organic solvent; and
subsequently forming of an aerosol stream by spraying from a nozzle for applying to a surface to be treated,
wherein the aerosol stream of liposomes is formed at an outlet upon spraying from an expanding part (8) of the mixing chamber, the expanding part being a divergent tube; and
wherein the mixing is accomplished in an ejector (3) comprising the mixing chamber by suction-induced introduction of the lipid component into the mixing chamber of the ejector by means of jet energy of the aqueous medium flowing out of an inlet nozzle (4) of the ejector, the aqueous medium flowing out creating rarefaction in a converging part (7) of the mixing chamber, the converging part being a convergent tube, with simultaneous dispersing and homogenizing in the expanding part of the mixing chamber, the mixing chamber made in the form of a Laval nozzle comprising the converging part and the expanding part.

2. The method for producing liposomal drugs in an aerosol stream according to claim 1, **characterized by** the aqueous medium and/or the lipid component comprising a biologically active substance.

3. A device for producing liposomal drugs in an aerosol stream, comprising:
a first vessel (1) comprising an aqueous medium;
a second vessel (2) comprising a lipid component, the lipid component being a solution of lipids in an organic solvent;
an ejector (3) in fluid communication with the first vessel and the second vessel, the ejector comprising a mixing chamber (6), a central inlet nozzle (4) and an outlet;
a pump (5) positioned between the first vessel and the ejector and adapted for feeding under pressure the aqueous medium from the first vessel into the mixing chamber of the ejector, wherein the mixing chamber is a Laval nozzle,
wherein the mixing chamber comprises a converging part(7), the converging part being a convergent tube, and an expanding part (8), the expanding part being a divergent tube, and is adapted for mixing the aqueous medium, fed under pressure into the mixing chamber, with the lipid component, by suction-induced introduction of the lipid component into the mixing chamber by means of jet energy of the aqueous medium flowing out of the inlet nozzle of the ejector,
wherein the mixing chamber is further adapted for the aqueous medium flowing out of the inlet nozzle creating rarefaction in the converging part of the mixing chamber, with simultaneous dispersing and homogenizing in the expanding part of the mixing chamber,
wherein an outlet of the expanding part of the mixing chamber serves as the outlet of the mixing chamber, the expanding part of the mixing chamber being adapted for forming an aerosol stream for applying to a surface to be treated, the outlet being a spray nozzle.

4. The device of claim 3, wherein the ejector (3) is in fluid communication with the first vessel (1) and the second vessel (2) via corresponding pipelines.

5. The device of claim 4, wherein the pipelines comprise corresponding control valves (9).

6. The device of claim 3, wherein the fluid communication between mixing chamber (3) and the second vessel (2) is free of a pump.

## Patentansprüche

1. Verfahren zur Herstellung liposomaler Zusammensetzungen in einem Aerosolstrom, umfassend:
das Mischen eines wässrigen Mediums (1), das unter Druck einer Mischkammer (6) zugeführt wird, mit einer Lipidkomponente (2), wobei die Lipidkomponente ein Lösung von Lipiden in einem organischen Lösungsmittel ist; und
das nachfolgende Bilden eines Aerosolstroms durch Versprühen aus einer Düse zum Auftragen auf eine zu behandelnde Oberfläche,
wobei der Aerosolstrom aus Liposomen an einem Austritt durch Versprühen aus einem sich erweiternden Teil (8) der Mischkammer gebildet wird, bei dem es sich um ein divergentes Rohr handelt; und
wobei das Mischen in einem die Mischkammer enthaltenden Ejektor (3) erfolgt durch Ansaugen der Lipidkomponente in die Mischkammer des Ejektors mittels der Strahlenergie des aus einer Eintrittsdüse (4) des Ejektors ausströmenden wässrigen Mediums, wobei das ausströmende wässrige Medium eine Verdünnung in einem konvergenten Teil (7) der Mischkammer bewirkt, bei dem es sich um ein konvergentes Rohr handelt, während im sich erweiternden Teil der Mischkammer eine Dispergierung und Homogenisierung erfolgt, wobei die in Form einer Lavaldüse ausgebildete Mischkammer den konvergenten und den erweiterten Teil umfasst.

2. Verfahren zur Herstellung liposomaler Zusammensetzungen in einem Aerosolstrom nach Anspruch 1, **dadurch gekennzeichnet, dass** das wässrige Medium und/oder die Lipidkomponente einen biologisch aktiven Stoff enthalten bzw. enthält.

3. Vorrichtung zur Herstellung liposomaler Zusammensetzungen in einem Aerosolstrom, enthaltend:
einen ersten Behälter (1) mit einem wässrigen Medium;
einen zweiten Behälter (2) mit einer Lipidkomponente, wobei die Lipidkomponente eine Lösung von Lipiden in einem organischen Lösungsmittel ist;
einen Ejektor (3) in fluidischer Verbindung mit dem ersten und dem zweiten Behälter, wobei der Ejektor eine Mischkammer (6), eine zentrale Eintrittsdüse (4) und einen Austritt aufweist;
eine zwischen dem ersten Behälter und dem Ejektor angeordnete Pumpe (5), die ausgebildet ist, das wässrige Medium unter Druck in den ersten Behälter zur Mischkammer des Ejektors zu führen,
wobei die Mischkammer eine Lavaldüse ist,
wobei die Mischkammer einen konvergenten Teil (7), bei dem es sich um ein konvergentes Rohr handelt, und einen sich erweiternden Teil (8) aufweist, bei dem es sich um ein divergentes Rohr handelt, und dazu ausgebildet ist, das unter Druck der Mischkammer zugeführte wässrige Medium mit der Lipidkomponente zu vermischen, indem die Lipidkomponente mittels der Strahlenergie des aus der Eintrittsdüse des Ejektors ausströmenden wässrigen Mediums in die Mischkammer eingesaugt wird,
wobei die Mischkammer weiter derart ausgebildet ist, dass das aus der Eintrittsdüse ausströmende wässrige Medium im konvergenten Teil der Mischkammer eine Verdünnung bewirkt, während im sich erweiternden Teil der Mischkammer gleichzeitig eine Dispergierung und Homogenisierung erfolgt,
wobei ein Austritt des sich erweiternden Teils der Mischkammer als Austritt der Mischkammer dient und der sich erweiternde Teil der Mischkammer dazu ausgebildet ist, einen Aerosolstrom zum Auftragen auf eine zu behandelnde Oberfläche zu bilden, und der Austritt eine Sprühdüse ist.

4. Vorrichtung nach Anspruch 3, wobei der Ejektor (3) mit dem ersten Behälter (1) und dem zweiten Behälter (2) über entsprechende Rohrleitungen in fluidischer Verbindung steht.

5. Vorrichtung nach Anspruch 4, wobei die Rohrleitungen jeweils Regelventile (9) aufweisen.

6. Vorrichtung nach Anspruch 3, wobei in der fluidischen Verbindung zwischen der Mischkammer (3) und dem zweiten Behälter (2) keine Pumpe vorhanden ist.

## Revendications

1. Procédé de production de préparations liposomales dans un jet d'aérosol, comprenant:
le mélange d'un milieu aqueux (1), alimenté sous pression à une chambre de mélange (6), avec un composant lipidique (2), le composant lipidique étant une solution de lipides dans un solvant organique; et
la formation subséquente d'un jet d'aérosol par pulvérisation à partir d'une buse pour son application sur une surface à traiter,
où le jet d'aérosol de liposomes est formé à un orifice de sortie par pulvérisation à partir d'une partie s'élargissante (8) de la chambre de mélange, la partie s'élargissante étant un tube divergent; et
où le mélange a lieu dans un éjecteur (3) comprenant la chambre de mélange par aspiration du composant lipidique dans la chambre de mélange de l'éjecteur au moyen de l'énergie du jet du milieu aqueux s'écoulant d'une buse d'admission (4) de l'éjecteur, le milieu aqueux qui s'écoule créant une raréfaction dans une partie convergente (7) de la chambre de mélange, la partie convergente étant un tube convergent, avec dispersion et homogénisation simultanées dans la partie élargie de la chambre de mélange, la chambre de mélange réalisée sous la forme d'une buse de Laval comprenant la partie convergente et la partie s'élargissante.

2. Procédé de production de préparations liposomales dans un jet d'aérosol selon la revendication 1, **caractérisé en ce que** le milieu aqueux et/ou le composant lipidique contiennent/contient une substance biologiquement active.

3. Dispositif pour la production de préparations liposomales dans un jet d'aérosol, comprenant:
un premier récipient (1) contenant un milieu aqueux;
un deuxième récipient (2) contenant un composant lipidique, le composant lipidique étant une solution de lipides dans un solvant organique;
un éjecteur (3) en communication fluidique avec le premier récipient et le deuxième récipient, ledit éjecteur comprenant une chambre de mélange (6), une buse centrale d'admission (4) et un orifice de sortie;
une pompe (5) disposée entre le premier récipient et l'éjecteur et adaptée à alimenter sous pression le milieu aqueux du premier récipient à la chambre de mélange de l'éjecteur,
où la chambre de mélange est une buse de Laval,
où la chambre de mélange comprend une partie convergente (7), la partie convergente étant un tube convergent, et une partie s'élargissante (8), la partie s'élargissante étant un tube divergent, et est adaptée à mélanger le milieu aqueux, alimenté sous pression à la chambre de mélange, avec le composant lipidique par aspiration du composant lipidique dans la chambre de mélange au moyen de l'énergie du jet du milieu aqueux s'écoulant de la buse d'admission de l'éjecteur,
où la chambre de mélange est en outre adaptée à ce que le milieu aqueux s'écoulant de la buse d'admission crée une raréfaction dans la partie convergente de la chambre de mélange, avec dispersion et homogénisation simultanées dans la partie élargie de la chambre de mélange,
où un orifice de sortie de la partie s'élargissante de la chambre de mélange sert d'orifice de sortie de la chambre de mélange, la partie s'élargissante de la chambre de mélange étant apte à former un jet d'aérosol destiné à être appliqué sur une surface à traiter, ledit orifice de sortie étant une buse de pulvérisation.

4. Dispositif selon la revendication 3, où l'éjecteur (3) est en communication fluidique avec le premier récipient (1) et le deuxième récipient (2) par des conduites correspondantes.

5. Dispositif selon la revendication 4, où les conduites comprennent des soupapes de réglage (9) respectives.

6. Dispositif selon la revendication 3, où la communication fluidique entre la chambre de mélange (3) et le deuxième récipient (2) ne comprend aucune pompe.
